# EUROPEAN PATENT APPLICATION

(11) **EP 2 444 519 A1**
(43) Date of publication of application: **25.04.2012**
(21) Application number: 09845053.9
(22) Date of filing: 08.10.2009
(51) Int. Cl.: C23C 16/27

(54) **DIAMOND-LIKE CARBON THIN FILM CONTAINING SILICON, PREPARATION METHOD THEREOF, AND USE THEREOF**

(71) Applicant: KIST Korea Institute of Science and Technology, Seongbuk-gu Seoul 136-791 (KR)
(72) Inventor: Moon,Myoung-Woon, Seoul 151-050 (KR); YI, Jin Woo, Seoul,136-120 (KR); Lee,Kwang Ryeol, Seoul,137-948 (KR); Kim,Hae-Ri, Seoul,142-100 (KR)
(74) Representative: Herden, Andreas F.
(86) International application number: PCT/KR2009/005765
(87) International publication number: WO 2011/043503

(57) **Abstract**

A silicon-incorporated diamond-like carbon thin film, a fabrication method thereof, and its use are disclosed. The silicon-incorporated diamond-like carbon thin film comprises a chemical bond between carbon and silicon atoms present on a surface of the silicon-incorporated diamond-like carbon thin film comprising silicon incorporated within and on the surface thereof with an atom providing hydrophilicity to the surface of the thin film on the surface of the thin film.

## Description

### FILE OF THE INVENTION

The present invention relates to a silicon-incorporated diamond-like carbon film, a fabrication method thereof, and its use.

### BACKGROUND OF THE INVENTION

Since a diamond-like carbon (DLC) thin film has a high hardness, lubrication, electric resistance and good abrasion resistance, has a smooth surface, and can be synthesized at a low temperature, it is a coating material used in various industrial fields. In addition, the DLC thin film has an excellent chemical stability of its surface, excellent biocompatibility and compatibility to the blood, not causing a side effect when it is in contact with cells or the like in vivo. Thus, it has been known to be easily applicable as a coating for a material for transplantation or cell cultivation, and accordingly, it has been attempted to use the same as a bio-coating such as a surface layer of an insertion or replacement material for a living body.

For example, a peripheral occlusive artery disease is a common disease diagnosed by about 3% of adults in their 40s and 50s, and about 20% of adults in their 70s by non-invasive diagnosis equipment. An interventional operation using a blood vessel stent in treating this disease has been widely used because it is simple and stable compared with a surgical operation, does not require general anesthesia, and has a high success rate. As the blood vessel stent, a bare stent, which is not coated, has been generally used, and thus, it requires that a stent wire, which comes into the inner wall of blood vessels, be surface-treated in order to improve biocompatibility. Furthermore, the stent for a blood vessel may induce an acute obstruction due to a blood clot formation immediately after the installation of the stent, and the stent itself may act as a traumatic element on the inner wall of blood vessels to induce an intimal hyperplasia, which causes restenosis problem. Thus, in order to increase the success rate of stent operation, it is required a surface treatment to restrain the coagulation of blood clot and a functional surface modification so as to render a drug release function for the direct delivery of the drug into blood vessels.

In order to restrain coagulation of blood clot and restenosis of blood vessel stent, research on coating a diamond-like carbon film onto the surface of the stent has been actively conducted. In particular, in order to prevent a leakage of metal ions from the stent material, a coating layer having an excellent corrosion resistance is required, and it is expected that the diamond-like carbon film meets such requirements. However, it has been reported that a pure diamond-like carbon thin film hardly exhibits coating effects. The reason is that the pure diamond-like carbon thin film does not have sufficient corrosion-resistance characteristics and blood compatibility.

In an effort to solve this problem, as a method for binding cells, organs or the like to the surface of biomaterials, in the convention technique, the surface of a silicon-containing DLC (Si-DLC) thin film is treated with plasma using oxygen or nitrogen so as to render hydrophilicity to the surface [Roy et al., Diamond and Related Materials, 16 (2007), 1732-1738]. Although the surface of the material which was treated as such has super-hydrophilicity, with the elapse of time, the surface of the material rapidly recovers hydrophobicity, i.e., the state before the surface treatment. This is called an aging effect, and because of this, the material has to be applied for a particular purpose such as bio-application or the like within a few hours after the surface treatment to render hydrophilicity.

A hydrophilic surface or super-hydrophilic surface having a good affinity with pure water has been continuously studied for the purpose of water harvesting, anti-fog, anti-bacteria or cell growth, or for the purpose of improving binding characteristics with other materials by modifying the characteristics of a material surface.

In order to form a hydrophilic or super-hydrophilic surface on the surface of a material, wet etching, ultraviolet/oxygen (UV/O) treatment, plasma/ion treatment, or the like, has been used. In particular, it has been known that a hydrophilic or super-hydrophilic surface can be obtained by increasing the roughness of the surface and adjusting surface chemical properties using a hydrophilic material. Implementation of hydrophilicity on the surfaces of various materials and thin films has been attempted, but surface hydrophilicity easily disappears. This is because that the hydrophilic surface has a relatively high surface energy, so it has a tendency to easily bind with water molecules or hydrocarbon molecules in the air so as to lower its surface energy, and when such binding occurs, the hydrophilicity disappears. As a result, the most hydrophilic or super-hydrophilic surfaces treated according to the conventional methods loses their hydrophilicity within a few hours or days. Therefore, researches for making the hydrophilic or super-hydrophilic characteristics maintained for a longer period of time have been variably conducted.

It has been known that the aging effect appears because the surface treated with oxygen or nitrogen plasma or the like has become an increased hydrophilicity, but it is thermodynamically instable and thus recovers its hydrophobicity [Roy et al., Diamond and Related Materials, 16 (2007), 1732-1738]. A technique for preventing the aging effect can be applied as a coating technique for restraining a mirror in a bathroom from being fogged, glasses from being fogged in the winter season, the vehicle glasses or the like from being fogged, as well as an application to the field requiring biological applications.

The recently developed method for fabricating a super-hydrophilic surface includes a method for depositing a material having a large number of nano-size pores, such as TiO₂ or the like, and a method for fabricating a hydrophilic surface using a mixture of nano-size particles such as TiO₂ particles, SiO₂ particles and the like in a proper ratio [FC Cebeci, Langmuir 22 (2006), 2856]. However, hydrophilicity of a surface fabricated with those methods does not last for a longer period of time. Therefore, maintaining the surface hydrophilicity for a long period of time is still a significant task.

### SUMMARY OF THE INVENTION

An object of the present invention is to improve corrosion resistance of a diamond-like carbon (DLC) thin film, and modify the surface of the DLC thin film having the improved corrosion resistance so as to adjust surface energy, thereby improving blood compatibility of the DLC thin film.

Another object of the present invention is to provide a method for semi-permanently maintaining hydrophilicity of the surface of the DLC thin film, corrosion resistance of which was improved, without an aging effect.

Still another object of the present invention is to provide a mass production method of a DLC thin film, the surface hydrophilicity of which is semi-permanently maintained.

In order to achieve the above objects, there is provided (1) a silicon-incorporated DLC thin film containing chemical bonds of carbon and silicon atoms present on a surface of the silicon-incorporated DLC thin film comprising silicon incorporated within and on the surface thereof with an atom (A) providing hydrophilicity to the surface of the thin film on the surface of the thin film.

In order to achieve the above objects, there is also provided (2) a material for the medical use, comprising the silicon-incorporated DLC thin film of (1).

In order to achieve the above objects, there is provided (3) a method for fabricating a silicon-incorporated DLC thin film, comprising: (a) forming a silicon-incorporated DLC thin film, wherein silicon atoms are incorporated within and on the surface of the DLC thin film, on a surface of a substrate; and (b) activating the surface of the thin film, followed by generating chemical bonds of carbon and silicon atoms present on the surface of the thin film with an atom (A) providing hydrophilicity to the surface of the thin film.

According to the present invention, a DLC thin film having an improved corrosion resistance and blood compatibility, and its fabrication method. Thus, because the thin film according to the present invention has excellent corrosion resistance and blood compatibility, it can be widely applied for treating surfaces of a insertion materials for a living body that is in contact with blood or a material for a treatment, such as a blood stent, a heart valve, a heart pump, an artificial blood vessel, a phathological laboratory material for restraining coagulation of blood, a blood storage container, and the like.

In addition, according to the present invention, a Si-DLC thin film having a nano-structure with semi-permanent super-hydrophilicity can be fabricated according to a simple and less-energy consuming method, and it is possible to make surfaces of various materials super-hydrophilic because the Si-DLC thin film having the super-hydrophilicity can be coated on the surface of any material,

### BRIEF DESCRIPTION OF THE DRAWINGS

Fig. 1 is a schematic view of RF-PACVD equipment used in the present invention;
Fig. 2 shows results of measuring contact angles with pure water in order to asses surface energy of each test sample when the surface of an Si-incorporated DLC thin film was plasma-treated with various reaction gases;
Fig. 3 shows results of measuring the ratio of an area of the surface of each test sample to which blood platelet is adhered when the surface of an Si-incorporated DLC thin film is plasma-treated with various reaction gases;
Fig. 4 is a SEM photograph of blood platelets adhered to the surface of the Si-incorporated DLC thin film which was not plasma-treated;
Fig. 5 is a SEM photograph of blood platelets adhered to the surface of the Si-incorporated DLC thin film which was plasma-treated;
Fig. 6 shows results of potentiodynamic polarization experiments of a substrate itself, a test sample obtained by coating a pure DLC thin film on the substrate, and a test sample obtained by coating an Si-incorporated DLC thin film on the substrate;
Fig. 7 shows results of measuring the behavior of blood platelet adhesion respectively to pure amorphous carbon and pure amorphous silicon whose surface was treated with oxygen plasma;
Fig Fig. 8(a) is a schematic view showing a process of ion beam treatment to an Si-DLC thin film according to the present invention, and Fig. 8(b) is an AFM image of the surface fabricated according to the process;
Fig. 9 shows optical microscope images for measuring wetting angles before ion beam treatment of the surfaces of materials deposited with a pure-DLC thin film (a) and a pure Si-DLC thin film (b);
Fig. 10 shows optical microscope images showing the comparison of wetting angles after the surface of the pure-DLC thin film was respectively treated with oxygen and nitrogen ion beams, in which (a) and (b) are images obtained after six hours and 21 days after the pure-DLC thin film was treated with N₂ ion beam, and (c) and (d) are images obtained after one day and twenty-two days after the pure-DLC thin film was treated with O₂ ion beam;
Fig. 11 shows optical microscope images showing the comparison of wetting angles after the surface of the Si-DLC thin film was treated respectively with oxygen and nitrogen ion beams, in which (a) and (b) are images respectively obtained six hours and 21 days after the pure-DLC thin film was treated with N₂ ion beam, and (c) and (d) are images obtained after one day and twenty-one days after the pure-DLC thin film was treated with O₂ ion beam;
Fig. 12 shows changes in wetting angles depending on time after the pure-DLC thin film surface and the Si-DLC thin film surface were respectively treated with oxygen and nitrogen ion beams;
Fig. 13 shows results of measuring the change in wetting angles depending on the content of silicon in the Si-DLC thin film, and wetting angles of the surface of a DLC thin film without Si and the surface of a thin film deposited only with amorphous Si for more than 20 days, in which a solid mark indicates results before the ion beam treatment and an open mark indicates results after the ion beam treatment; and
Fig. 14(a) to (d) are AFM images of surface roughness of four test samples obtained by treating the surfaces of a pure-DLC thin film and a Si-DLC thin film which were respectively treated with oxygen and nitrogen ion beams, and (e) is the profile of a representative cross section before and after the surface of each test sample was treated.

### DETAILED DESCRIPTION OF THE INVENTION

The present invention relates to a silicon-incorporated DLC thin film containing chemical bonds of carbon and silicon atoms present on a surface of the silicon-incorporated DLC thin film comprising silicon incorporated within and on the surface thereof with an atom (A) providing hydrophilicity to the surface of the thin film on the surface of the thin film.

In the present invention, instead of a pure DLC thin film, a silicon-incorporated DLC thin film having excellent corrosion resistance is used. The Si-incorporated DLC thin film comprises silicon atoms in the form of clusters in size of a few to scores of nanometers which are distributed within and on the surface thereof, by which the strain specific to the DLC thin film can be reduced and durability and biocompatibility can be improved compared with the DLC thin film.

The content of the silicon in the Si-incorporated DLC thin film preferably ranges from 0.5 at.% to 17 at.%. If the silicon content is less than 0.5 at.%, sufficient corrosion resistance characteristics are not obtained and the hydrophilicity of the surface tends to easily disappear, while if the silicon content exceeds 17 at.%, the size of SiC clusters within the thin film is so large that the mechanical and chemical characteristics are deteriorated.

In addition, the silicon content in the Si-DLC thin film deposited on the surface of a substrate is adjusted, and carbon and silicon atoms present on the surface of the thin film are chemically bonded with an atom (A) providing hydrophilicity to the surface of the thin film, so as to modify the surface of the thin film, thereby making the surface of the thin film hydrophilic and maintaining super-hydrophilicity for a long period of time. In order to make the super-hydrophilicity of the surface of the thin film being maintained for a long period of time, the silicon content in the Si-DLC thin film is preferably 1.0 at.% to 2.5 at.%. In this case, when the thin film surface is activated, the surface roughness becomes 10 nm to 20 nm, and the super-hydrophilicity of the surface is maintained for a long period time.

Because the surface of the silicon-incorporated DLC thin film according to the present invention is modified, its biocompatibility and blood compatibility are excellent. The surface modification is resulted from surface activation and chemical bonds of carbon and silicon atoms present on the surface of the thin film with the atoms providing hydrophilicity to the surface of the thin film. In the present invention, the atoms providing hydrophilicity to the surface of the thin film are oxygen and/or nitrogen atom. In general, hydrophilicity is expressed as a water contact angle. According to the present invention, the contact angle of the modified surface of the thin film exceeds 0° but it is below 50°, preferably, exceeds 0° but it is below 20°, for the sake of blood compatibility.

In addition, the present invention relates to a material for medical use, comprising the silicon-incorporated DLC thin film. The material medical use may include a blood stent, a heart valve, a heart pump, an artificial blood vessel, a phathological laboratory material for restraining coagulation of blood, a blood storage container, and the like.

In addition, the present invention relates to a method for fabricating a silicon-incorporated DLC thin film, comprising: (a) forming a DLC thin film, in which silicon atoms are incorporated within and on the surface of the DLC thin film, on a surface of a substrate; and (b) activating the surface of the thin film, followed by generating chemical bonds of carbon and silicon atoms present on the surface of the thin film with the atoms (A) providing hydrophilicity to the surface of the thin film.

In step (a), the Si-incorporated DLC thin film may be formed by a method selected from general thin film formation methods, for example, any of a plasma chemical vapor deposition (CVD), a plasma synthesis, a sputtering synthesis, a self-filtering arc synthesis and an ion beam deposition, or any combination thereof.

In case of forming a thin film with a plasma CVD, a carbon precursor, e.g., benzene, acetylene or methane, and a precursor gas for silicon, e.g., silane (SiH₄), are put in a plasma CVD container, and then plasma-treated, or silicon may be supplied by sputtering while performing plasma synthesis with a carbon precursor. In case of forming a thin film by plasma CVD, a bias voltage is preferably in the range of -100V to -800V, and the pressure inside the device is preferably in the range of 0.5 Pa to 10 Pa.

In case of forming a thin film with plasma CVD, the carbon precursor and the precursor gas for silicon may be used together, or silicon may be sputtered while synthesizing a DLC thin film with carbon plasma.

The thickness of the thin film formed in step (a) may be in the range of from 0.001 µm to 10 µm.

The substrate may be, for example, a blood stent, a heart valve, a heart pump, an artificial blood vessel, a phathological laboratory material for restraining coagulation of blood, a blood storage container, and the like.

In step (b), the thin film surface may be activated by various methods. For example, the surface of the Si-incorporated DLC thin film may be activated by irradiating RF plasma, DC plasma, plasma beam or ion beam to the surface of the thin film.

In case of using plasma to activate the surface of the thin film, the pressure inside the chamber preferably ranges from 0.1 Pa to 10 Pa, a bias voltage may range from -100 V to -800 V. In case of using ion beam to activate the surface of the thin film, the pressure within the chamber preferably ranges from 1.0×10⁻⁷ Pa to 10 Pa and the voltage may preferably range from 100 V to 50 kV.

In step (b), when the thin film surface is activated, atoms (A) of a reactive gas decomposed by plasma or ion beam are chemically bonded with carbon or silicon atoms, to form bonds between C and A atoms, and bonds between Si and A atoms. In the present invention, in order to make the modified thin film surface hydrophilic, preferably, oxygen or nitrogen is used as the reactive gas. It was discovered that Si-A bonds, rather than C-A bonds, contributes more to the hydrophilicity of the thin film surface (See Example 1) .

### EXAMPLES

The present invention will now be described in detail through Examples. However, these examples are merely illustrative and the scope of the present invention is not limited thereto.

### Example 1: Deposition of thin film and surface treatment of the thin film using plasma

### 1. Fabrication of Si-incorporated DLC thin film

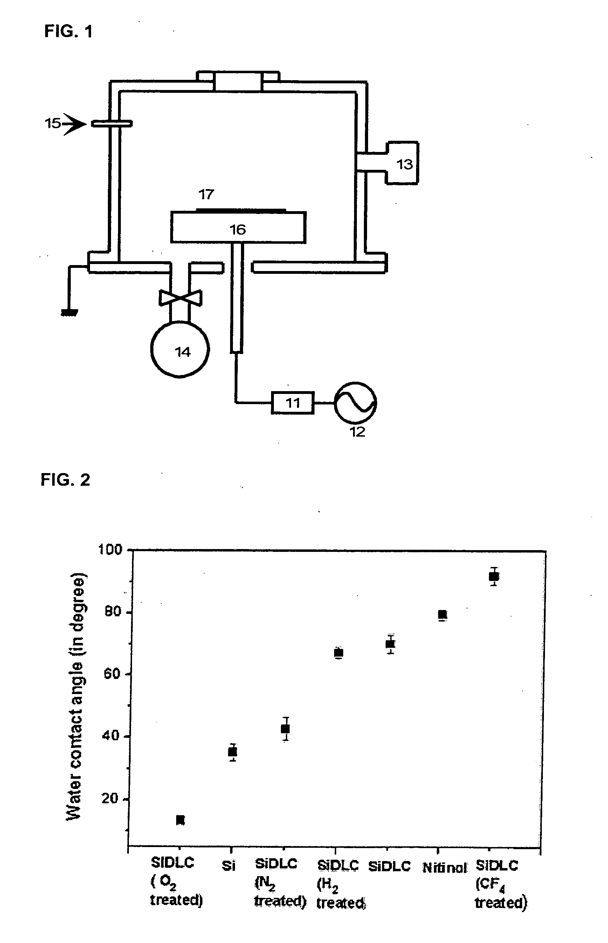

A Si-incorporated DLC thin film was formed on the surface of a substrate 17 using an RF-PACVD equipment illustrated in Fig. 1. A detailed procedure is as follows:

The substrate 17 was cleansed with in the order of trichloroethylene (TCE), acetone and methanol for 20 minutes, respectively, using a ultrasonic cleanser and then installed on an electrode 16 inside a vacuum reactive chamber which is cooled with water. The inside of the reactive chamber was maintained in a vacuum at 1.0 x 10⁻⁵ Torr using a vacuum pump 14. An argon gas was introduced into the chamber through a gas inlet 15, and then the substrate 17 was dry-cleansed with plasma which was generated by applying radiowave power of -400 V to the electrode 16. Reference numeral 11 denotes an RF matching unit, 12 denotes an RF generator, and 13 denotes a baratron gauge.

Next, benzene (C₆H₆) gas and silane (SiH₄) were introduced using a mass flow controller (MFC) into the interior of the chamber in a ratio such that the Si content in a thin film to be formed was 1.2 at.% to 2.5 at%, and then a radiowave power was applied to form an Si-DAC thin film.

### 2. Surface modification of Si-incorporated thin film

The Si-DLC thin film obtained in 1 above was put into the reactor illustrated in Fig. 1 and then its surface was treated with oxygen, nitrogen, hydrogen and CF₄ plasma for 10 minutes, respectively. In order to analyze surface energy of the surface-treated test sample, contact angles with pure water were measured, and the results obtained thereby are shown in Fig. 2. The bias voltage in the plasma treatment was -400 V and the pressure was 1.33 Pa.

As shown in Fig. 2, surfaces of various characteristics of surfaces having a high hydrophilicity to a high hydrophobicity were formed depending on the gases used for the plasma treatment. According to X-ray photoelectron spectroscopy (XPS) analysis results, it was discovered that in the surface of the test sample treated with oxygen plasma, Si-O and C-O bonds were formed with high concentrations, thereby increasing polar components, by which the surface became hydrophilic. Meanwhile, the surface treated with CF₄ plasma became hydrophobic because a large number of C-F bonds were present on the surface. Such a change resulted from a rapid decrease in polar components due to the C-F bonds on the surface (See Table 1)

**Table 1**

| **Thin film** | **Chemical bonds present on the surface (XPS analysis)** | **Water contact angle (degree)** | **Surface energy (nJ/cm²)** | | |
|---|---|---|---|---|---|
| | | | **Dispersive component** | **Polar component** | **Total** |
| SiDLC | C-C, Si-C | 70.1±3.0 | 28.6±5.2 | 11.1±3.7 | 39.7±8.9 |
| SiDLC (H₂ treated) | C-C, Si-H | 67.2±1.8 | 29.9±3.5 | 12.3±2.5 | 42.1±6.0 |
| SiDLC (CF₄ treated) | C-C, Si-C, -CFn, C-CF | 92.1±2.6 | 29.9±3.6 | 1.9±1.1 | 31.8±2.5 |
| SiDLC (N₂ treated) | C-C, Si-H, C-N | 42.7±3.7 | 26.1±3.2 | 30.3±4.7 | 56.4±1.7 |
| SiDLC (O₂ treated) | C-C, C-O, Si-H, Si-O, O-H | 13.4±1.3 | 18.0±0.3 | 53.1±0.8 | 71.0±1.1 |

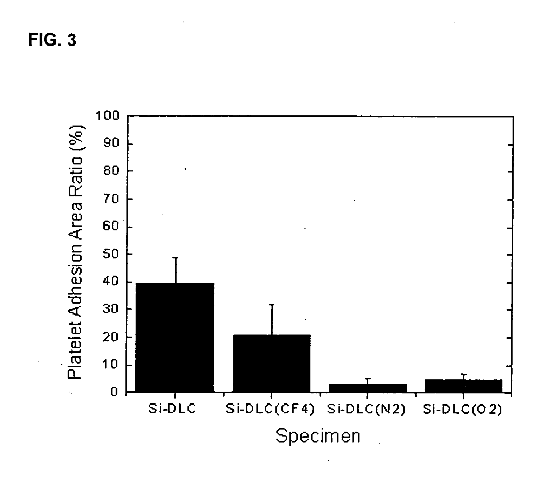

In order to determine blood compatibility of the test samples, the test samples were immersed in a blood platelet-concentrated plasma (concentration of blood platelet: 3.0 x 10⁸/ml) obtained from blood of a healthy person for sixty minutes, and then taken out and washed, and thereafter, the ratio of areas of the test samples to which blood platelet was adhered was measured. The results are shown in Fig. 3. Gases used for plasma treatment were indicated in the parentheses of a horizontal axis. Compared with a thin film whose surface was not treated, the surface-treated thin film had less amount of blood platelet adhered thereto. In particular, in case of the thin films respectively treated with nitrogen and oxygen plasma, adhesion of blood platelet was remarkably reduced.

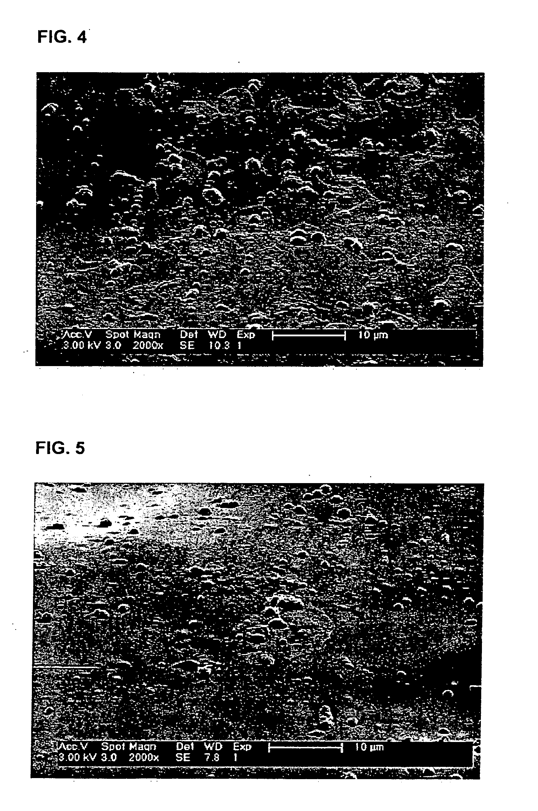

Fig. 4 shows the shape of blood platelet adhered to the Si-incorporated DLC thin film whose surface was not treated. It is noted that a large amount of blood platelets are adhered, pseudopodia is formed on the most blood platelets or the blood platelets are spread on the surface of the thin film. In comparison, Fig. 5 shows that a blood platelet-adhered area of the Si-incorporated DLC thin film which was treated with oxygen plasma is significantly small, and most of the adhered blood platelets remain inactivated. This results indicate that blood compatibility of the Si-incorporated DLC thin film was considerably increased by the oxygen plasma treatment.

### 3. Comparison of corrosion resistance characteristics of pure DLC thin film and Si-incorporated DLC thin film

In order to determine the corrosion resistance characteristics of the Si-incorporated DLC thin film and the pure DLC thin film, a Si-incorporated DLC thin film and a pure DLC thin film were respectively coated on a Ti-6Al-4V substrate, which is commonly used as a bio-material, at a bias voltage of -400 V, and potentiodynamic polarization test was performed. In this case, the Si content of the Si-incorporated DLC thin film was 2 at.%.

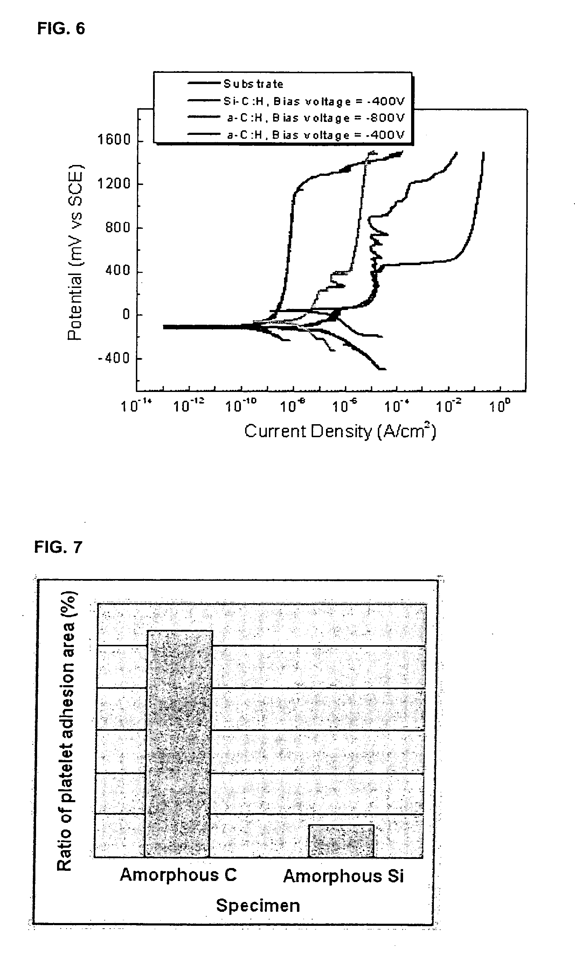

Fig. 6 shows that a passivation film was formed on all of the Ti-6Al-4V substrate, the test sample coated with a pure DLC thin film on the Ti-6Al-4V substrate, and the test sample coated with a Si-incorporated DLC thin film on the Ti-6Al-4V substrate. However, in case of the Ti-6Al-4V substrate, as the potential increases, the passivation film was destroyed at 500 mV and current density was sharply increased, while in case of the test sample coated with the pure DLC thin film, a very unstable passivity behavior appeared and then the passivation film was destroyed at a potential of 800 mV or higher. In comparison, in case of the test sample coated with the Si-incorporated DLC thin film, a corrosion current density was the lowest and showed very stable passive state behavior. These results show that the corrosion resistance of the Si-incorporated DLC thin film is excellent.

### 4. Comparison of contribution of Si-O and C-O bonds to hydrophilicity

Fig. 7 shows the results which compare blood platelet absorption degrees of the test sample coated with pure amorphous Si thin film and treated with oxygen-plasma, and a test sample coated with pure amorphous carbon thin film and treated with oxygen plasma. Each test sample has either Si-O bonds or C-O bonds. Compared with the carbon thin film treated with oxygen plasma, the Si thin film treated with oxygen plasma had significantly reduced adsorption of blood platelets, which means that Si-O bonds on the surface of the thin film greatly contribute to the improvement of blood compatibility. As a result, it was discovered that the significant increase of the blood compatibility when the thin film was treated with oxygen plasma resulted from Si-O bonds present on the surface of the thin film.

### Example 2: Thin film deposition and surface treatment using ion beam

### 1. Fabrication of DLC thin film

Before deposition, a substrate was cleansed for 15 minutes under 0.49 Pa and at -400 V with an argon ion beam. In order to enhance an absorption of Si-DLC thin film, amorphous silicon (a-Si) was deposited as an initial buffer layer between a DLC thin film and the substrate.

The DLC thin film and the Si-DLC thin film were respectively deposited on a P type Si (100) substrate using hybrid ion beam equipment. An voltage of the equipment 1000 V and a deposition pressure was 1.33 Pa. Benzene was used as a carbon source, and a diluted silane gas was used as a Si source (SiH₄/H₂=10:90). The thickness of the thin film was adjusted to be 0.55±0.01 µm. The thickness of the thin film was measured with alpha step profilometer. The Si content of the thin film was adjusted to range from 0 at.% to 4.88 at.% and determined with Rutherford backscattering spectroscopy (RBS).

Next, the deposited DLC thin film and Si-DLC thin film were respectively treated with nitrogen and oxygen ion beams. The pressure in the chamber during the ion beam treatment was 1.33 Pa, the voltage was 1000V, and the treatment time was 10 minutes. Since etching speed was 24 nm/min, it can be anticipated that the thin film was etched to have a thickness of 240 nm.

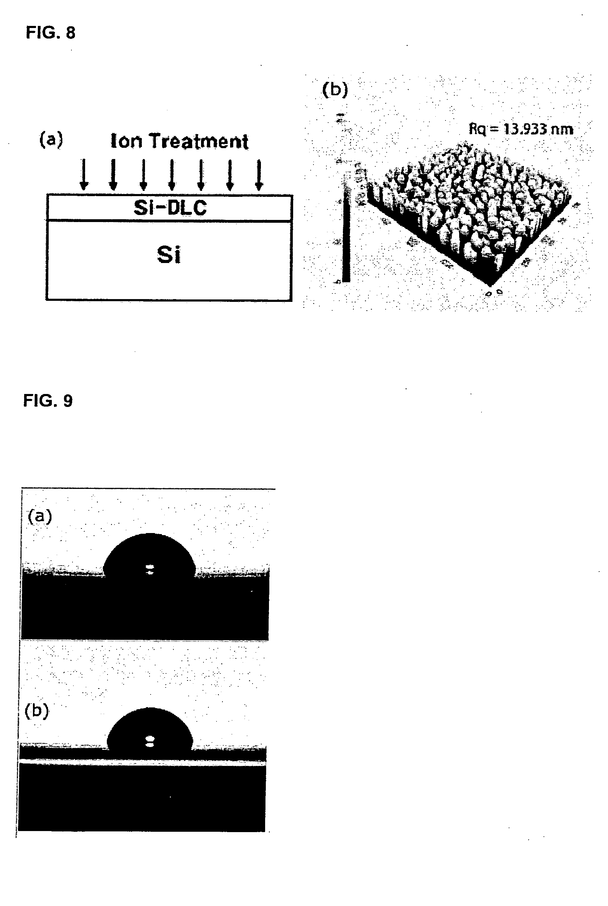

Fig. 8(b) shows that the roughness of the Si-DLC thin film surface increased by ion beam treatment. In particular, it was discovered that the roughness was maximized when the Si content was 1.0 at.% to 2.66 at.%. After the ion beam treatment was completed, the thin film was exposed in the air at room temperature, and the temperature was maintained at 20°C to 25°C and moisture was maintained at 60% to 70%.

### 2. Examination of thin film characteristics.

### (1) Measurement of wetting angle

After ion beam treatment, a wetting angle of each test sample was measured with distilled water over 20 days. After dust of the surface of each sample was blown out with nitrogen gas, and 5µl of distilled water (pure water drops) was dropped lightly to the surface of each test sample and wetting angles were measured. In order to precisely observe the wetting angle, the measured portion was indicated after measurement of the wetting angle, so that the wetting angle of the same portions of the surface of each test sample could not be measured again. This is because if the wetting angle is measured again at the portion of the surface contaminated with water, an accurate measurement cannot be made. In order to measure the wetting angle, NRL Contact Angle Goniometer was used. A baseline of the substrate was adjusted, pure water drops were lightly dropped, the angle measured by turning a goniometer was read, and an image of the wetting angle of the pure water drops was captured.

### (2) Surface analysis

A surface roughness of 2µm × 2µm area was determined using Autoprobe CP research system (Thermo Microscope Inc, USA) as Atomic Force Microscope (AFM) equipment. Root Mean Square (RMS) value was adopted as the surface roughness.

### 3. Effects of surface treatment with N₂ and O₂ ion beams

As shown in Figs. 9(a) and 9(b), the wetting angles of the DLC thin film and the Si-DLC thin film (the Si content was 2.66 at.%) which were not been ion beam treated were 76° which were similar to each other, and in this condition, the wetting angles were maintained regardless of the elapse of time.

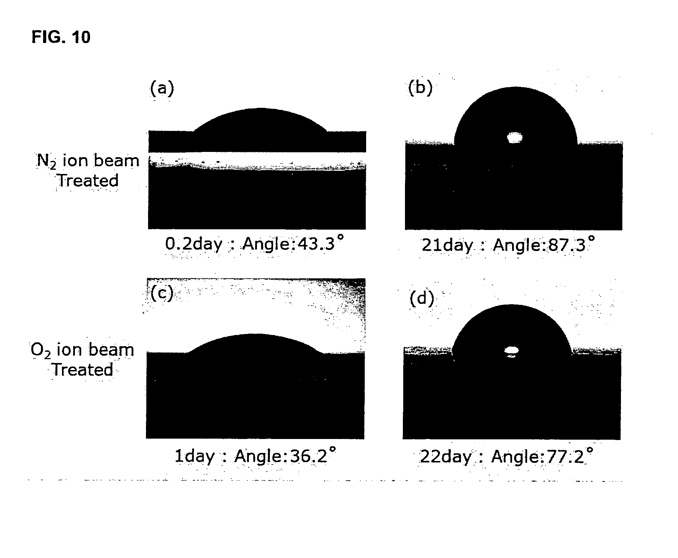

However, Figs. 10(a) to 10(d) show an aging effect appearing when the DLC thin film was respectively treated with O₂ and N₂ ion beams. In particular, the wetting angle measured after six hours from the treatment of the DLC thin film surface with N₂ ion beam was 43.3° (Fig. 10(a)), and the wetting angle measured after 21 days from the treatment of the DLC thin film surface with N₂ ion beam was 86.3° (Fig. 10(b)). Meanwhile, the wetting angle measured one day after the DLC thin film surface was treated with O₂ ion beam was 36.2° (Fig. 10(c)), and the wetting angle measured 22 days after the DLC thin film surface was treated with O₂ ion beam was 77.2° (Fig. 10(d)). These results show that although the surface of the pure DLC thin film is treated with oxygen or nitrogen ion beam, it loses hydrophilicity as the wetting angle increases.

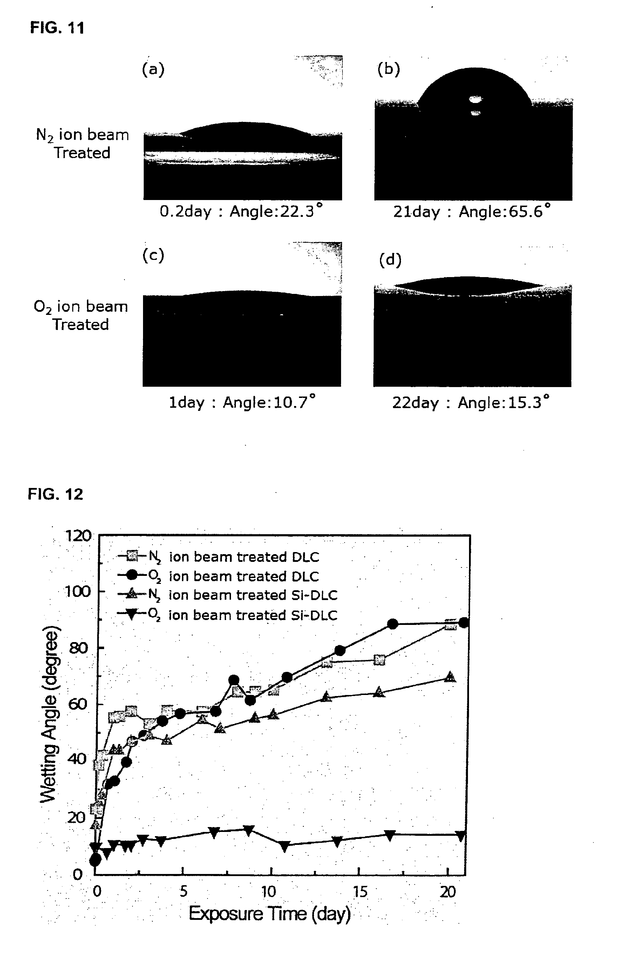

Fig. 11 shows that the pure water wetting angle on the surface of the Si-DLC thin film has different behavior from that of the DLC thin film. In detail, the wetting angle measured immediately after the Si-DLC thin film surface was treated with N₂ ion beam was 22.3° (Fig. 11(a)), the wetting angle measured 21 days after the Si-DLC thin film surface was treated with N₂ ion beam was 65.6° (Fig. 11(b)), the wetting angle measured immediately after the Si-DLC thin film surface was treated with O₂ ion beam was 10.7° (Fig. 11(c)), and the wetting angle measured 21 days after the Si-DLC thin film surface was treated with O₂ ion beam was 15.3° (Fig. 11(d)).

Namely, after 20 days, the wetting angle of the surface of the Si-DLC thin film treated with N₂ ion beam was about 60°, and thus, it recovered hydrophobicity, whereas the wetting angle was about 15.3° for the surface of the Si-DLC thin film treated with O₂ ion beam, and thus, the aging effect rarely occurred.

Fig. 12 shows continuously measured wetting angles for more than 20 days after the pure-DLC thin film and the Si-DLC thin film were surface-treated. Fig. 12 shows that in case of DLC thin film, the test sample treated with N₂ ion beam has a faster initial aging speed than the test sample treated with O₂ ion beam, but they have the similar wetting angle 20 days after the surface treatment. However, in case of the Si-DLC thin film, although it was treated with O₂ in the same manner, the hydrophilicity of its surface lasted longer than that of the DLC thin film.

When comparing four test samples obtained by surface treatment of a pure-DLC thin film and a Si-DLC thin film surface with oxygen or nitrogen ion beam, the aging effect most rapidly occurred in an initial stage when the DLC thin film surface was treated with N₂ ion beam. However, in case of the DLC thin film, there is a mere difference in the initial aging speed but the same degree of aging effect occurred after about five days. In comparison, in case of the Si-DLC thin film, its aging speed is slow compared with the DLC thin film, and in particular, when the Si-DLC thin film surface was treated with O₂ ion beam, the wetting angle of about 15°C was maintained even after 20 days.

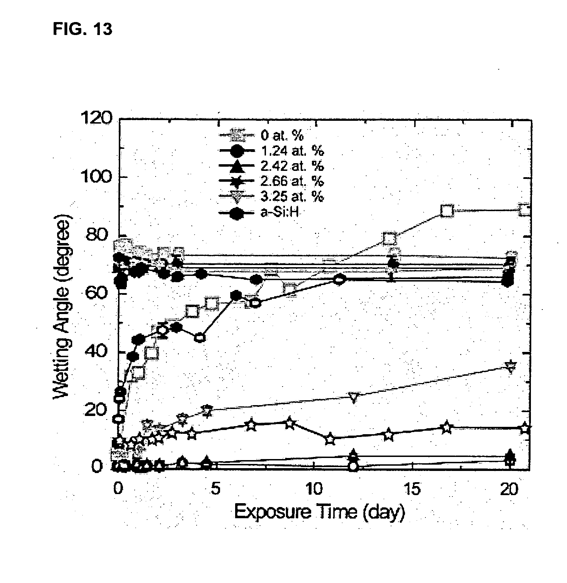

Fig. 13 shows the results of observing changes of wetting angles of the Si-DLC thin film surface over time when the atomic percentage (at.%) of the Si of the Si-DLC thin film was changed. When the Si content of the Si-DLC thin film was 1.0 at.% to 2.0 at.% and the Si-DLC thin film was not treated with O₂ ion beam, the wetting angle of about 75° was uniformly maintained for 20 days. However, when test samples having Si contents of 1.24 at.% and 2.42 at.%, respectively, were treated with O₂ ion beam, their wetting angle was 8° six hours after it was exposed in the air, and although the wetting angle was gradually increased, it was less than 15° even after 20 days, which indicates that the hydrophilicity (or super-hydrophilicity) was maintained. In comparison, when the silicon content in the Si-DLC thin film was 2.66 at.% and 3.25 at.%, respectively, the initial low wetting angles were increased to 14° and 27°, respectively. This result shows that when the Si content increases, the aging effect gradually occurs. In particular, when the Si content was 100 at.%, i.e., in case of the surface deposited only with amorphous hydrogenated silicon (a-Si:H) thin film, the aging effect rapidly occurred after the surface treatment, which is similar to the aging effect occurring from the general silicon surface.

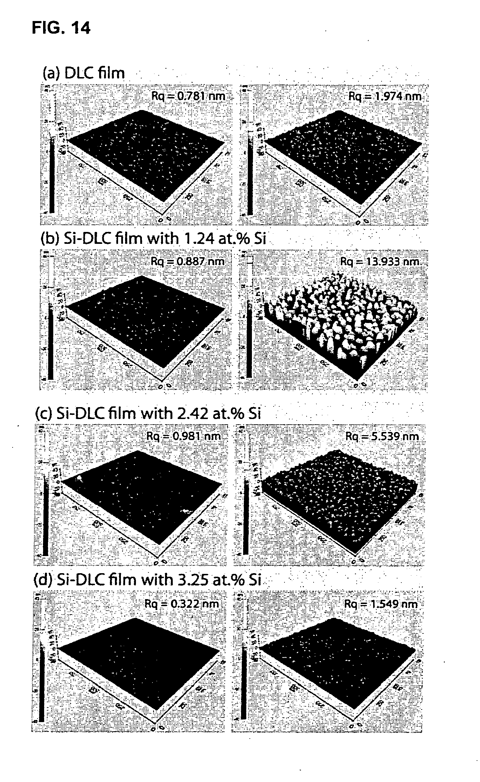

With reference to the AFM images of Figs. 14(a) to 14(d), it is noted that in case that the Si-DLC thin film was treated with 02 ion beam, its surface roughness value is high when the Si content of the thin film was 1.24 at.% and 2.42 at.%. As shown in Table 2 below, in case that the Si-DLC thin film was treated with oxygen ion beam, the Si content before the ion beam treatment were 1.24 at.% and 2.42 at.%, respectively, whereas the Si content of the surface were measured by about 17% and 20%, respectively, after the oxygen ion treatment. It is understood that this was resulted from the fact that carbon on the surface of the thin film was etched by the oxygen ion beam treatment, and thus, the Si present in the thin film was exposed, thereby increasing the Si content at the surface.

**[Table 2]**

| | | C(at.%) | N(at.%) | O(at.%) | Si(at.%) |
|---|---|---|---|---|---|
| DLC film | as dep. | 93.55 | 0.23 | 6.22 | 0 |
| | as mod. | 72.15 | 0.72 | 26.28 | 0.85 |
| | at 1^{st} day | 73.37 | 0.71 | 25.24 | 0.68 |
| | at 20^{th} day | 70.14 | 1.42 | 27.6 | 0.84 |
| 1.24 at.% | as dep. | 91.37 | 0 | 7.39 | 1.24 |
| Si-DLC | as mod. | 26 | 0.71 | 55.6 | 17.68 |
| | at 1^{st} day | 27.06 | 0.72 | 55.18 | 17.03 |
| | at 20^{th} day | 30.03 | 0.89 | 52.33 | 16.75 |
| 2.42 at.% | as dep. | 89.08 | 0 | 7.6 | 2.42 |
| Si-DLC | as mod. | 20.47 | 0.53 | 59.02 | 19.99 |
| | at 1^{st} day | 21.02 | 0.62 | 58.52 | 19.85 |
| | at 20^{th} day | 22.77 | 0.9 | 56.69 | 19.65 |
| 3.25 | at.% as dep. | 87.36 | 0 | 9.4 | 3.25 |
| Si-DLC | as mod. | 24.93 | 0.63 | 56.23 | 18.21 |
| | at 1^{st} day | 25.53 | 0.67 | 55.83 | 17.96 |
| | at 20^{th} day | 28.4 | 1.33 | 52.91 | 17.36 |

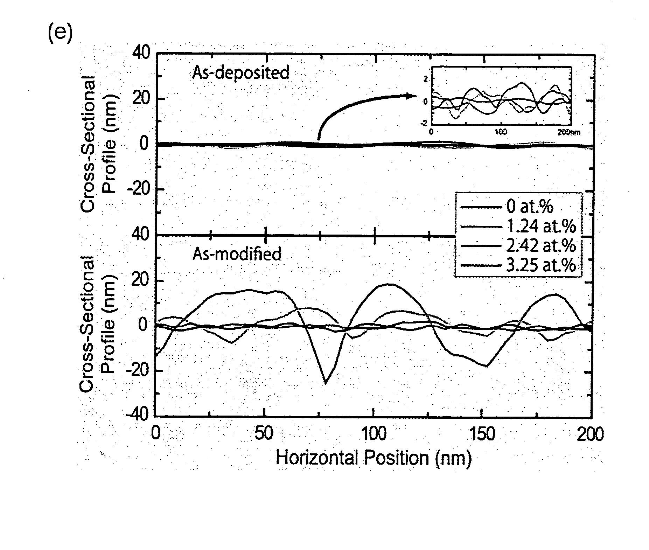

As shown in Fig. 14 and Table 2, it is understood that the continuation of the hydrophilicity of the surface of the Si-DLC thin film according to the present invention for a long period of time attributes to the formation of roughness in nano scale on the surface of the thin film by the oxygen ion beam treatment and the increase in the Si content on the surface. This is because when the Si-DLC thin film is treated with O₂ ion beam, carbon as a main component of the thin film is etched, while silicon remains and forms nano-particles having the roughness in nano size on the surface of the thin film. Since such nano-particles are largely made of Si component, Si-O bonds are increased during the O₂ ion beam treatment. The fact that the hydrophilicity was resulted from the formation of the bonds of polar components on the surface of the thin film was identified through X-ray photoelectron spectroscopy (XPS) analysis.

As described above, when the DLC thin film and the Si-DLC thin film are treated with N₂ or O₂ ion beam, all the test samples exhibited hydrophilicity immediately after they were treated, but the aging effect that hydrophobicity is recovered with the elapse of time was observed. However, when the surface of the Si-DLC thin film was treated with the O₂ ion beam, the hydrophilicity was maintained for more than 20 days. The analysis results obtained with AFM shows that the surface roughness was maximized when the Si-DLC thin film was treated with O₂, and the surface roughness in nano size served to increase the hydrophilicity and maintain the hydrophilicity for a long time. When the Si-DLC thin film was treated with O₂, carbon as a main component of the thin film was etched, while Si remained to form the surface roughness in nano size on the surface of the thin film. The surface having the roughness in nano size is largely made of Si, generating Si-O bonds during the O₂ ion beam treatment. According to the XPS analysis, it was discovered that chemical bonds of polar components are formed on the surface of the thin film, and such bonds provide hydrophilicity to the surface of the thin film and makes the hydrophilicity maintained semi-permanently.

As the present invention may be embodied in several forms without departing from the characteristics thereof, it should also be understood that the above-described examples are not limited by any of the details of the foregoing description, unless otherwise specified, but rather should be construed broadly within its scope as defined in the appended claims, and therefore all changes and modifications that fall within the metes and bounds of the claims, or equivalents of such metes and bounds are therefore intended to be embraced by the appended claims.

## Claims

1. A silicon-incorporated diamond-like carbon thin film containing chemical bonds of carbon and silicon atoms present on a surface of the silicon-incorporated diamond-like carbon thin film comprising silicon incorporated within and on the surface thereof with an atom (A) providing hydrophilicity to the surface of the thin film on the surface of the thin film.

2. The silicon-incorporated DLC thin film of claim 1, wherein silicon content in the silicon-incorporated diamond-like carbon thin film ranges from 0.5 at.% to 17 at.%.

3. The silicon-incorporated diamond-like carbon thin film of claim 1, wherein silicon content in the silicon-incorporated diamond-like carbon thin film ranges from 1.0 at.% to 2.5 at.%.

4. The silicon-incorporated diamond-like carbon thin film of claim 3, wherein the silicon-incorporated diamond-like carbon thin film has a surface roughness of from 10 nm to 20 nm.

5. The silicon-incorporated diamond-like carbon thin film of claim 1, wherein the atom (A) providing hydrophilicity to the surface of the thin film is oxygen or nitrogen atom.

6. The silicon-incorporated diamond-like carbon thin film of claim 1, wherein the atom (A) is oxygen atom, and Si-O bonds on the surface of the thin film range from 30% to 60%.

7. The silicon-incorporated diamond-like carbon thin film of claim 1, wherein a contact angle of surface of the silicon-incorporated diamond-like carbon thin film exceeds 0° but is not more than 50°.

8. The silicon-incorporated diamond-like carbon thin film of claim 1, wherein a contact angle of surface of the silicon-incorporated diamond-like carbon thin film exceeds 0° but is not more than 20°.

9. A material for the medical use, comprising the silicon-incorporated diamond-like carbon thin film of claim 1.

10. The material of claim 9, wherein the material is a blood stent, a heart valve, a heart pump, an artificial blood vessel, a phathological laboratory material for restraining coagulation of blood, or a blood storage container.

11. The material of claim 9, used for growth of cells or an organ of a living body.

12. A method for fabricating a silicon-incorporated diamond-like carbon thin film, comprising:
(a) forming a silicon-incorporated diamond-like thin film, wherein silicon atoms are incorporated within and on the surface of the diamond-like thin film, on a surface of a substrate; and
(b) activating the surface of the silicon-incorporated diamond-like thin film, followed by generating chemical bonds of carbon and silicon atoms present on the surface of the thin film with an atom (A) providing hydrophilicity to the surface of the thin film.

13. The method of claim 12, wherein in step (a), the silicon-incorporated diamond-like thin film is formed with a method selected from the group consisting of a plasma chemical vapor deposition, a plasma synthesis, a sputtering synthesis, a self-filtering arc synthesis or an ion beam deposition, or any combination thereof.

14. The method of claim 12, wherein silicon content in the silicon-incorporated diamond-like carbon thin film formed in step (a) ranges from 0.5 at.% to 17 at.%.

15. The method of claim 12, wherein silicon content in the silicon-incorporated diamond-like carbon thin film formed in step (a) ranges from 1.0 at.% to 2.5 at.%.

16. The method of claim 12, wherein in step (b), the surface of the silicon-incorporated diamond-like thin film is activated by plasma or ion beam treatment.

17. The method of claim 16, wherein the pressure inside the chamber in the plasma treatment ranges from 0.1 Pa to 10 Pa, and a bias voltage ranges from -100V to -800V.

18. The method of claim 16, wherein the pressure in the ion beam treatment ranges from 1.0×10⁻⁷ Pa to 10 Pa, and the voltage ranges from 100V to 50kV.

19. The method of claim 12, wherein the surface of the silicon-incorporated diamond-like thin film obtained in step (b) has a roughness of from 10 nm to 20 nm.

20. The method of claim 12, wherein the atom (A) providing hydrophilicity to the surface of the silicon-incorporated diamond-like thin film is oxygen or nitrogen atom.

21. The method of claim 12, wherein a contact angle of the surface of the silicon-incorporated diamond-like thin film obtained in step (b) exceeds 0° but is not more than 50°.

22. The method of claim 12, wherein a contact angle of the surface of the silicon-incorporated diamond-like thin film obtained in step (b) exceeds 0° but is not more than 20°.

23. The method of claim 12, wherein the substrate is a blood stent, a heart valve, a heart pump, an artificial blood vessel, a phathological laboratory material for restraining coagulation of blood, or a blood storage container.

24. The method of claim 12, wherein the substrate is a glass, mirror or silicon substrate.

25. A method for improving blood compatibility of a silicon-incorporated diamond-like carbon thin film, comprising treating a surface of the silicon-incorporated diamond-like carbon thin film, wherein silicon is present within and on the surface of the thin film, with an oxygen or nitrogen plasma or ion beam, to generate Si-N or Si-O bonds on the surface of the silicon-incorporated diamond-like carbon thin film.

26. A method for providing hydrophilicity to a silicon-incorporated diamond-like carbon thin film, comprising treating a surface of the silicon-incorporated diamond-like carbon thin film, wherein silicon is present within and on the surface of the thin film is treated with an oxygen or nitrogen plasma or ion beam, to generate Si-N or Si-O bonds on the surface of the silicon-incorporated diamond-like carbon thin film.

27. The method of claim 25 or 26, wherein the silicon content in the silicon-incorporated diamond-like carbon thin film ranges from 0.5 at.% to 17 at.%.

28. A method for semi-permanently maintaining hydrophilicity of a silicon-incorporated diamond-like carbon thin film, comprising treating a surface of the silicon-incorporated diamond-like carbon thin film, wherein silicon is present within and on the surface of the thin film with oxygen plasma to generate Si-O bonds on the surface of the thin film.

29. The method of claim 28, wherein the silicon content in the silicon-incorporated diamond-like carbon thin film ranges from 1.0 at.% to 2.5 at.%.

30. A method for preventing surface of mirror, glass or silicon from being fogged by forming the silicon-incorporated diamond-like carbon thin film of claim 1 on the surface thereof.
